# EUROPEAN PATENT SPECIFICATION

(11) **EP 4 562 336 B1**
(45) Date of publication and mention of the grant of the patent: **11.11.2026**
(21) Application number: 23750545.8
(22) Date of filing: 21.07.2023
(51) Int. Cl.: F16N 21/00, F16N 7/38, F16N 19/00, F16N 27/00, A61M 39/10, F16L 55/00

(54) **ARRANGEMENT AND METHOD FOR LUBRICATION**
ANORDNUNG UND VERFAHREN ZUR SCHMIERUNG
AGENCEMENT ET PROCÉDÉ DE LUBRIFICATION

(30) Priority: 29.07.2022 SE 2250935
(43) Date of publication of application: 04.06.2025
(73) Proprietor: Interlinked AB, 111 39 Stockholm (SE)
(72) Inventor: AXELSSON, Robert, 563 91 GRÄNNA (SE); BEJHED, Rebecca, 753 37 Uppsala (SE); HEDBECK, Katarina, 111 60 Stockholm (SE)
(74) Representative: AWA Sweden AB
(86) International application number: PCT/EP2023/070241
(87) International publication number: WO 2024/022959

(56) References cited:
- EP-A1- 3 030 761
- WO-A1-2018/087153
- CN-A- 111 636 943
- DE-A1- 102014 017 250

## Description

### FIELD OF THE INVENTION

The present invention generally relates to the field of lubrication arrangements and methods. More specifically, the present invention relates to an arrangement and a method for lubrication of one or more elements or components.

### BACKGROUND OF THE INVENTION

There are numerous arrangements, devices, equipment, actuators, etc., in many fields of technology, which benefit from lubrication. The lubricant may be applied to components, parts, areas, etc., of the arrangements, which come into contact during operation of the arrangements, in order to reduce friction. Consequently, the lubrication may prolong the arrangements' service lives and/or avoid breakdowns or malfunctions thereof. CN111636943 A describes a known lubrication arrangement.

According to an example, medical devices and/or arrangements may also benefit from the lubrication thereof. During patient treatments, there may be a need of transferring one or more fluids (blood, blood products, one or more medicines, etc.) to and/or from a patient. As the fluid often is supplied via an element (e.g. a needle, cannula, catheter, trocar, or the like) inserted into the patient during treatments of this kind, it is desirable that the element remains relatively fixed after insertion into the patient. Involuntarily displacements and/or movements of the element after insertion into the patient may arise in case the patient or any medical staff accidentally pulls a tube connected to the element. Furthermore, stumbling accidents of the patient and/or medical staff over a tube connected to an element may also lead to element displacements. It will be appreciated that displacements of this kind of an element may not only be painful for the patient, but may also lead to consequences of the treatment if the transferring of fluid due to the element's displacement is not performed correctly. Furthermore, it should be noted that pulling an element inserted into a patient with force could damage the vessel of a patient. Furthermore, for a patient with a compromised immune system, a wounded vessel could lead to a serious infection. In addition, in case of an accidental pulling of a tube for a transfer of a fluid to a patient, it is desirable that any leakage of the fluid is mitigated. For example, if the infusion liquid is toxic, any leakage from a broken tube may be especially hazardous.

WO2018/087153 A1 discloses a coupling device which overcomes the problems as mentioned above, as the coupling device enables a transfer of fluid through the coupling device which is sealed to the outside environment when the coupling device is connected, and a leak-free interruption of a transfer of a fluid in case the coupling device is disconnected. More specifically, upon connection of the coupling device, first and second sealing elements firstly provide a sealing upon abutment with each other. Thereafter, a transfer of fluid is enabled by the tube portion of the coupling device, projecting through (penetrating) the first and second sealing elements. Upon disconnection of the coupling device, which disconnection may be triggered by a pulling force applied to the third housing and the second housing of the coupling device, the second housing may be displaced from its second (retracted) position to its first (extracted) position. Consequently, this results in a retraction of the tube portion from its position through the first and second sealing elements such that the second housing and the third housing subsequently become sealed again by the first and second sealing elements, respectively.

There is a desire to improve lubrication arrangements and/or methods in the prior art of many fields of technology. More specifically regarding medical devices, there is a wish to even further improve, or maintain during use, the leak-free properties of these devices, and e.g. of coupling devices of the kind according to WO2018/087153 A1. Hence, it is desirable to provide an efficient and convenient lubrication of one or more elements, parts, components, or the like, of any kind of arrangement, device, equipment, actuator, or the like, such as a medical coupling device, for an improved operation and/or service life thereof.

### SUMMARY OF THE INVENTION

It is an object of the present invention to improve, or maintain during use, the operation and/or service life of arrangements and/or devices via efficient and convenient lubrication of one or more parts, elements, components, or the like, of the arrangements and/or devices.

This and other objects are achieved by providing an arrangement and a method having the features in the independent claims. Preferred embodiments are defined in the dependent claims.

Hence, according to a first aspect of the present invention, there is provided an arrangement for lubrication of at least one component. The arrangement comprises a container arranged to contain a lubricant, and a conduit for the lubricant, wherein the conduit is connected to the container at a first end portion of the conduit. The arrangement further comprises a pump connected to a second end portion of the conduit, wherein the pump, upon operation, is configured to generate an underpressure in the conduit. The conduit comprises a conduit section between the first end portion and the second end portion of the conduit, wherein the conduit section comprises an opening arranged to receive at least a part of the at least one component, a first passage for the lubricant, wherein the first passage is fluidically connected to the opening and arranged upstream of the opening, and a second passage for the lubricant, wherein the second passage is fluidically connected to the opening and arranged downstream of the opening. Upon a reception of the at least one component to the opening, the lubricant, via the underpressure, is transferred through the conduit to the opening via the first passage, and from the opening via the second passage, for lubrication of the at least one component.

According to a second aspect of the present invention, there is provided a method for lubrication of at least one component, via an arrangement comprising a container arranged to contain a lubricant, a conduit for the lubricant, wherein the conduit is connected to the container at a first end portion of the conduit, a pump connected to a second end portion of the conduit, wherein the pump, upon operation, is configured to generate an underpressure in the conduit, wherein the conduit comprises a conduit section between the first end portion and the second end portion of the conduit. The conduit section comprises an opening arranged to receive at least a part of the at least one component, a first passage for the lubricant, wherein the first passage is fluidically connected to the opening and arranged upstream of the opening, and a second passage for the lubricant, wherein the second passage is fluidically connected to the opening and arranged downstream of the opening. The method comprises the step of arranging the at least one component at the opening, whereby the lubricant, via the underpressure, is transferred through the conduit to the opening via the first passage, and from the opening via the second passage, for lubrication of the at least one component.

Thus, the present invention is based on the idea of providing an arrangement for lubrication purposes, which is arranged or configured for lubrication of one or more components or elements of any arrangements, devices, equipment, actuators, etc. For example, the arrangement may be arranged or configured for lubrication of one or more elements or components of a medical coupling device configured to transfer a fluid. One or more components may be received by the opening of the conduit section of the arrangement, whereby the arrangement provides a lubrication of the component(s) in a convenient, efficient and/or fast manner, consequently leading to an improved operation after the lubrication process.

The present invention is advantageous in that the arrangement provides excellent sealing properties at reception of the component(s) for the lubrication operation. Hence, the arrangement achieves a reliable sealing upon reception of the component(s) in the conduit section opening, thereby ensuring that there is no leakage, or at least a minimized and/or mitigated leakage, of lubricant during and/or after application to the component(s).

The present invention is further advantageous in that the arrangement may receive and/or accommodate components of different shapes and/or sizes for lubrication thereof. Hence, the present invention provides a versatile arrangement for lubrication purposes. It will be appreciated that one or more parts or sections (e.g. the conduit section) of the arrangement may be 3D-printed, which even further contributes to the convenience and versatility of the arrangement.

The present invention is further advantageous in that the arrangement is fast in its lubrication application. For example, a component may be lubricated in seconds, or even in the time region of one second, by the arrangement. This time-efficiency of the arrangement is highly advantageous regarding several aspects. For example, in case of a medical device, a minimized changeover time of the coupling device when the coupling device is taken out of service for lubrication thereof, a minimized time for personnel (e.g. nurse(s), doctor(s), etc.) for lubricating the coupling device component(s) by the arrangement, etc., may be achieved. Consequently, the arrangement is able to save time and/or cost for staff or personnel concerning the lubrication service of the coupling device.

The present invention is further advantageous in that the arrangement is convenient and intuitive in its use, as the component(s) may be arranged in the opening of the arrangement. Consequently, the arrangement does not require any particular and/or complex education and/or training before usage.

The present invention is further advantageous in that the arrangement achieves a passive operation concerning the application of lubricant. More specifically, as the pump of the arrangement is configured to generate an underpressure in the conduit, the reception of the component(s) to the opening of the conduit section triggers the transfer of the lubricant to the opening of the conduit section, thereby achieving a fast and/or automatic lubrication process. Hence, the lubrication process as provided by the arrangement is particularly convenient in its operation, and the arrangement may not need any (further) elements or devices for initiating and/or performing the lubrication.

The arrangement is arranged for lubrication of at least one component. By the term "component", it is here meant substantially any element, part, section, element, or the like. For example, the component(s) may be component(s) of any arrangement, device, equipment, actuator, or the like. According to a specific example, the component(s) may be component(s) of a (medical) coupling device, wherein the coupling device is configured to transfer a fluid. Hence, the arrangement is arranged or configured for lubrication of component(s) of substantially any kind, wherein medical coupling devices for fluid transfer constitute examples thereof. In such a case, the coupling device may be of the kind comprising first and second connector elements configured to matingly connect, wherein the first connector element comprises a first sealing element and a tube portion, and wherein the second connector element comprises a second tube and a second sealing element. In a non-connected state of the coupling device, the first sealing element and the second sealing elements seal the respective second tube from the first tube. In a connected state of the coupling device, the tube portion projects through the first and second sealing elements, for fluid connection between the tube portion of the first connector element and the second connector element for enabling a transfer of fluid through the coupling device.

The arrangement comprises a container arranged to contain a lubricant. By "container", it is here meant substantially any container, canister, holder, vessel, tank, or the like. By the term "lubricant", it is here meant substantially any fluid capable of reducing friction, such as an (silicon) oil, fluid grease, etc.

The arrangement further comprises a conduit for the lubricant, wherein the conduit is connected to the container at a first end portion of the conduit, and a pump connected to a second end portion of the conduit, wherein the pump, upon operation, is configured to generate an underpressure in the conduit. The conduit comprises a conduit section between the first end portion and the second end portion of the conduit, wherein the conduit section comprises an opening arranged to receive at least a part of the at least one component. Hence, the conduit section is or constitutes a segment or a part of the conduit which comprises an opening or receiving section which is arranged or configured to receive at least a part of the component(s) which is (are) to be subjected to lubrication by the arrangement.

The conduit section further comprises a first passage for the lubricant, wherein the first passage is fluidically connected to the opening and arranged upstream of the opening. Hence, lubricant transported in the conduit from the container to the opening of the conduit section is arranged to pass through the second passage before being transported (supplied) to the opening.

The conduit section further comprises a second passage for the lubricant, wherein the second passage is fluidically connected to the opening and arranged downstream of the opening. Hence, lubricant transported (away) from the opening in the conduit is arranged to pass through the second passage.

Upon a reception of the at least one component to the opening, the lubricant, via the underpressure, is transferred through the conduit to the opening via the first passage, and from the opening via the second passage, for lubrication of the at least one component. Hence, the opening for reception and/or accommodation of the component(s) is provided between the first and second passages of the conduit for the lubricant.

According to an embodiment of the present invention, the conduit section may comprise an outer wall and an inner wall defining the first passage, wherein the outer wall defines the opening of the conduit section. The present embodiment is advantageous in that the outer wall may be adapted to and/or formed for the form and/or size of the component(s) which is (are) to be lubricated.

According to an embodiment of the present invention, a cross-section of the opening of the conduit section may have one of a circular shape and a rectangular shape. Hence, a cross-section of the opening of the conduit section for receiving the component(s) may be circular or rectangular (e.g. square). The present embodiment is advantageous in that the conduit section may easily, conveniently and/or efficiently receive the component(s) for lubrication.

According to an embodiment of the present invention, the conduit section, towards the opening thereof, has a funnel shape. Hence, the cross-section of the conduit section may increase towards the opening such that the conduit section, at least by a part thereof, may have a funnel shape. The present embodiment is advantageous in that the opening of the arrangement may conveniently and easily receive (at least a part of) the component(s) for lubrication.

According to an embodiment of the present invention, the arrangement may be arranged for lubrication of at least one component constituting a tube, wherein the second passage, via the opening, is arranged to matingly receive at least a part of the tube. Hence, the second passage of the conduit section of the arrangement may be arranged to matingly receive at least a part of a tube (e.g. of a coupling device), wherein the tube, for example, may be arranged or configured to project through (first and second) sealing elements of the coupling device for enabling a transfer of fluid through the coupling device. The present embodiment is advantageous in that the tube may be conveniently and intuitively inserted into the receiving opening for an application of lubricant to the tube. The present embodiment is further advantageous in that the arrangement may achieve a particularly reliable sealing in case of insertion of the tube into the second passage, thereby ensuring that there is no (or at least a mitigated) leakage of lubricant during and/or after application. The present embodiment is particularly advantageous in case of a tube of a coupling device, as the coupling device comprises one or more sealing elements that the tube of the coupling device is arranged or configured to penetrate upon connection of the coupling device (and analogously, through which sealing element(s) the tube may be withdrawn upon disconnections of the coupling device), as the lubricated tube of the coupling device thereby may glide with less friction through the sealing element(s). Consequently, this leads to reduced wear of the tube and/or sealing element(s), and an improved operation and/or service life of the device, such as a coupling device.

According to an example of the present invention, the first passage and the second passage may be separated by a wall portion. The present embodiment is advantageous in that conduit section is relatively non-complex and convenient in its design.

According to an embodiment of the present invention, the conduit section may comprise a sealing at a peripheral portion of the conduit section defining the opening, for a sealing of the reception of the at least one component to the opening. Hence, the sealing (e.g. in form of a ring) may ensure a leak-free contact between the opening and the component(s). The present embodiment is advantageous in that the arrangement may receive component(s) for lubrication in a sealed manner, thereby avoiding or minimizing any leakage of lubricant during operation of the arrangement.

According to an embodiment of the present invention, the pump may be a vacuum pump. The present embodiment is advantageous in that the vacuum pump may efficiently provide the underpressure in the conduit for transporting, transferring or sucking the lubricant from the container, through the conduit, to the conduit section and away from the conduit section in the arrangement.

According to the present invention, the arrangement further comprises a regulator mechanism for regulating the supply of lubricant from the container to the conduit. This is advantageous in that the arrangement may easily and conveniently adapt the amount of lubricant to the component(s) to be lubricated. For example, in case of (a) relatively large (area of the) component(s) for lubrication and/or if it is determined that a relatively large amount of lubricant is needed and/or desired for the specific component(s) and/or purposes thereof, the arrangement may provide a relatively large amount of lubricant by the regulator mechanism. In contrast, in case of (a) relatively small (area of the) component(s) for lubrication and/or if it is determined that a relatively small amount of lubricant is needed and/or desired for the specific component(s) and/or purposes thereof, the arrangement may provide a relatively small amount of lubricant. Hence, the present embodiment provides an even further augmented versatility with respect to the lubrication operation.

According to an embodiment of the present invention, the regulator mechanism may comprise an outer tube at least partially enclosing the first end portion of the conduit, wherein a portion of the outer tube extends into the container. The regulator mechanism may further comprise a floater arrangement arranged in the container and within the portion of the outer tube. The floater arrangement comprises a floater, and a sealing abutment for the floater, wherein the sealing abutment defines a space of the portion of the outer tube, wherein the outer tube comprises at least one aperture allowing a passage of ambient air outside the outer tube to the space of the portion of the outer tube. The floater arrangement is operable between a first state of the floater arrangement, wherein the floater is separated from the sealing abutment, allowing a supply of lubricant from the container to the space of the portion of the outer tube, and a second state of the floater arrangement, caused by a reception of the at least one component to the opening and the underpressure in the conduit, wherein the floater abuts the sealing abutment, impeding a supply of lubricant from the container to the space of the portion of the outer tube. In the second state, the floater arrangement defines a predetermined amount of lubricant in the space of the portion of the outer tube, and the at least one aperture allows ambient air to transfer the predetermined amount of lubricant to the conduit for lubrication of the at least one component. Hence, the regulator mechanism of the arrangement provides a two-state (i.e. first and second states) operation, wherein the regulator mechanism, in the first state, determines or provides a predetermined amount of lubricant before application, and in the second state transfers this amount of lubricant to the conduit section for application to the component(s). The regulator mechanism may hereby alternate between the first and second states for subsequent applications of lubrication dosages. The present embodiment is advantageous in that the regulator mechanism may provide lubrication to the opening of the conduit section, in which component(s) is (are) provided, in a repeatable, convenient and efficient manner.

According to an embodiment of the present invention, the outer tube may be adjustable with respect to the container for an adjustment of the predetermined amount of lubricant in the space of the portion of the outer tube. It will be appreciated that the size and/or form of the component(s) may need different and/or specific amounts of lubricant, and the present embodiment conveniently provides this desired and/or needed lubricant amount by its adjustability of the outer tube with respect to the container. The present embodiment hereby achieves a customized provision of lubricant, which may lead to savings of cost and/or avoidance of an excess of supplied lubricant, which consequently may lead to an even further mitigation of leakage of lubricant.

According to an embodiment of the present invention, the arrangement may further comprise a control unit connected to the regulator mechanism, wherein the control unit is configured to operate the regulator mechanism based on time control. Hence, the control unit may be configured to operate one or more functions of the regulator mechanism according to one of more (cycle) times, time intervals, etc., for the distribution of lubricant. The present embodiment is advantageous in that the reliability of the regulator mechanism may be improved even further.

According to an embodiment of the present invention, the arrangement may be arranged for lubrication of at least one component of a coupling device, wherein the coupling device is configured to transfer a fluid.

According to an embodiment of the present invention, there is provided a lubrication system, comprising a coupling device configured to transfer a fluid, and an arrangement according to any one of the preceding embodiments for lubrication of at least one component of the coupling device. For example, the coupling device may be of the kind comprising a first connector element, which in turn comprises a first sealing element and a tube portion, and a second connector element comprising a second sealing element. **In** a non-connected state of the coupling device, the first sealing element and the second sealing elements may provide a sealing of the coupling device. **In** a connected state of the coupling device, the tube portion may project through the first and second sealing elements, for enabling a transfer of fluid through the coupling device. The coupling device is advantageous in that its connection and reconnection (if detached or disconnected) is performed in an easy, convenient and efficient manner. Notably, the first and second connector elements of the coupling device are connectable via an easy and convenient plug operation, which may not need any screwing or twisting. For example, in case a tube connected to a coupling device is pulled apart, it may be desirable to be able to reinstate the (infusion) therapy again as quickly as possible. The coupling device meets this demand, as the (re)connection of the coupling device may be performed quickly and intuitively due to the innovative configuration of the coupling device. It will be appreciated that the ability of the coupling device to mitigate any leakage of fluid is advantageous for reasons of safety. For example, in case the coupling device is provided for a transfer of a toxic liquid, any leakage from the coupling system may be particularly hazardous. Hence, the coupling device may significantly increase the safety of medical staff and/or patients. The coupling device further provides a convenient detachment (disconnection) of the coupling device. For example, a pulling of the (tube connected to the) coupling device, e.g. by a patient and/or medical staff, may detach the first and second connector elements of the coupling device from each other. It will be appreciated that it may be desirable that (medical) tubes commonly used at hospitals, nursing homes, clinics, etc., are equipped with such a coupling device, as it may constitute "weak link(s)" of the tube(s). Hence, if one or more tubes is (are) connected between a patient and a source (e.g. an infusion pump or bag) and further comprises a coupling device as described, the coupling device may constitute the "weak link(s)" of the tube(s) where the tube(s) may be "cut off" as a result of a pulling of the tube(s). The coupling device is further advantageous in that it is relatively inexpensive to manufacture and is easily assembled. Consequently, the coupling device may primarily be designed for single use, i.e. the coupling device may for example be used for one patient and one therapy (e.g. infusion). The present embodiment is particularly advantageous for coupling devices of this kind, as the lubricated tube of the coupling device may glide with less friction through the (first and/or second) sealing element(s). Consequently, this leads to reduced wear of the tube and/or sealing element(s), and an improved operation and/or service life of the coupling device.

Further objectives of, features of, and advantages with, the present invention will become apparent when studying the following detailed disclosure, the drawings and the appended claims. Those skilled in the art will realize that different features of the present invention can be combined to create embodiments other than those described in the following.

### BRIEF DESCRIPTION OF THE DRAWINGS

This and other aspects of the present invention will now be described in more detail, with reference to the appended drawings showing embodiment(s) of the invention.
Fig. 1 is a schematic view of a part of an arrangement according to an exemplifying embodiment of the present invention,
Figs. 2-4 are schematic views of a cross-section of an arrangement according to exemplifying embodiments of the present invention,
Fig. 5 is a schematic view of a cross-section of a part of an arrangement according to an exemplifying embodiment of the present invention, and
Fig. 6 is a schematic view of a cross-section of a conduit section 400 of an arrangement according to an exemplifying embodiment of the present invention.

### DETAILED DESCRIPTION

It will be appreciated that the present invention is related to substantially any arrangements, devices, equipment, actuators, etc., which may benefit from lubrication. In the following, however, a coupling device is described as an example and/or for an improved understanding of the invention.

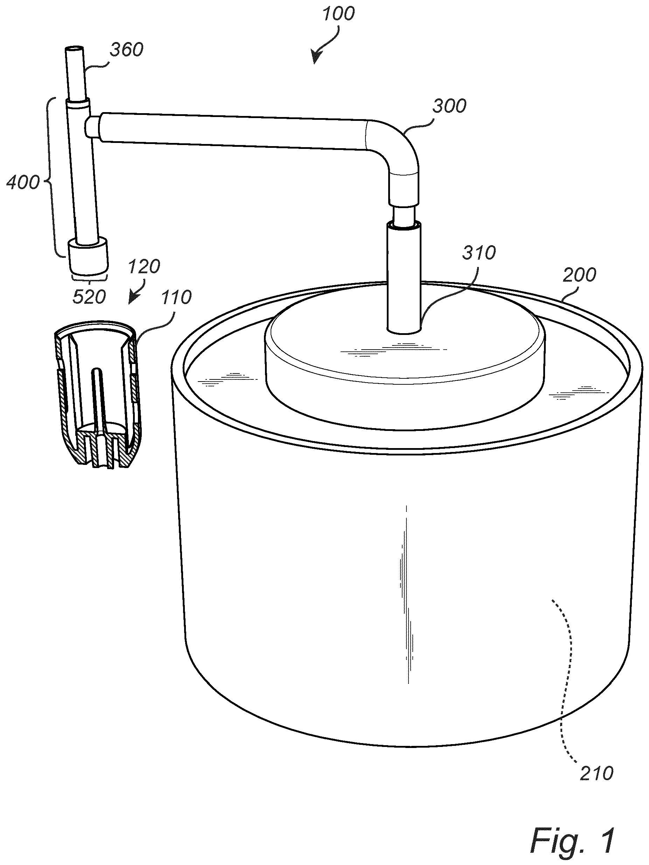

Fig. 1 is a schematic view of a part of an arrangement 100 according to an exemplifying embodiment of the present invention. The arrangement 100 is arranged for lubrication of at least one component 110 of a coupling device 120 arranged to transfer a fluid. It should be noted that the coupling device 120 may be substantially any kind of coupling device 120 (or part thereof), and that the (part of the) coupling device 120 in Fig. 1, which here is depicted in cross-section, is for describing and/or exemplifying purposes only. The arrangement 100 comprises a container 200 arranged to contain a lubricant 210. Here, the container 200 is exemplified in the form of a (cylinder-shaped) cistern, but it should be noted that the container 200 may be of substantially any form and/or size. The lubricant 210 may be a fluid capable of reducing friction and is preferably a silicon oil. The arrangement 100 further comprises a conduit 300 for the lubricant 210. The conduit 300 may have substantially any form, length and/or size. The conduit 300 is connected to the container 200 at a first end portion 310 of the conduit 300. The arrangement 100 further comprises a conduit section 400 between the first end portion 310 and a second end portion 360 of the conduit 300. The conduit section 400 comprises an opening 520 arranged to receive at least a part of the component(s) 110 of the coupling device 120. Upon a reception of the one or more components 110 of the coupling device 120 to the opening 520, the lubricant 210 is transferred through the conduit 300 to the opening 520 for lubrication of the component(s) 110.

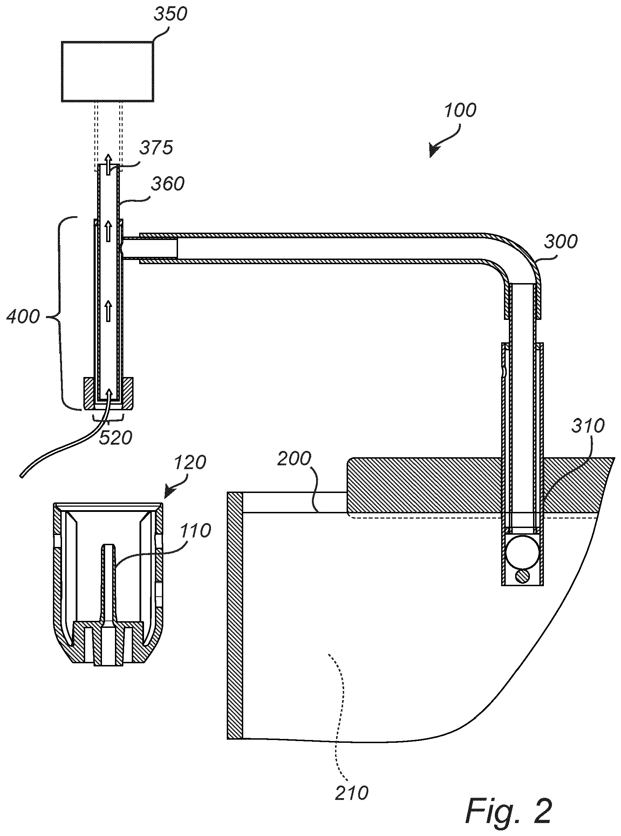

Fig. 2 is a schematic view of a cross-section of an arrangement 100 according to an exemplifying embodiment of the present invention. It will be appreciated that Fig. 2 has many features in common with Fig. 1, and it is hereby referred to Fig. 1 and the associated text for an increased understanding of the arrangement 100.

Analogously with Fig. 1, the arrangement 100 in Fig. 2 comprises a container 200 arranged to contain a lubricant 210. The arrangement 100 further comprises a conduit 300 for the lubricant 210, wherein the conduit 300 is connected to the container 200 at a first end portion 310 of the conduit 300. The arrangement 100 further comprises a pump 350, which merely is schematically indicated. The pump 350, which may be a vacuum pump 350, is connected to a second end portion 360 of the conduit 300. Upon operation, the pump 350 is configured to generate an underpressure in the conduit 300. The arrangement 100 further comprises a conduit section 400 between the first end portion 310 and the second end portion 360 of the conduit 300. The conduit section 400 comprises an opening 520 arranged to receive at least a part of the component(s) 110 of the coupling device 120.

At the state of the arrangement 100 shown in Fig. 2, the coupling device 120 is separated from the conduit section 400 of the arrangement 100, and the pump 350 is configured to generate a flow 375 through the opening 520 of the conduit section 400 towards the second end portion 360 of the conduit 300.

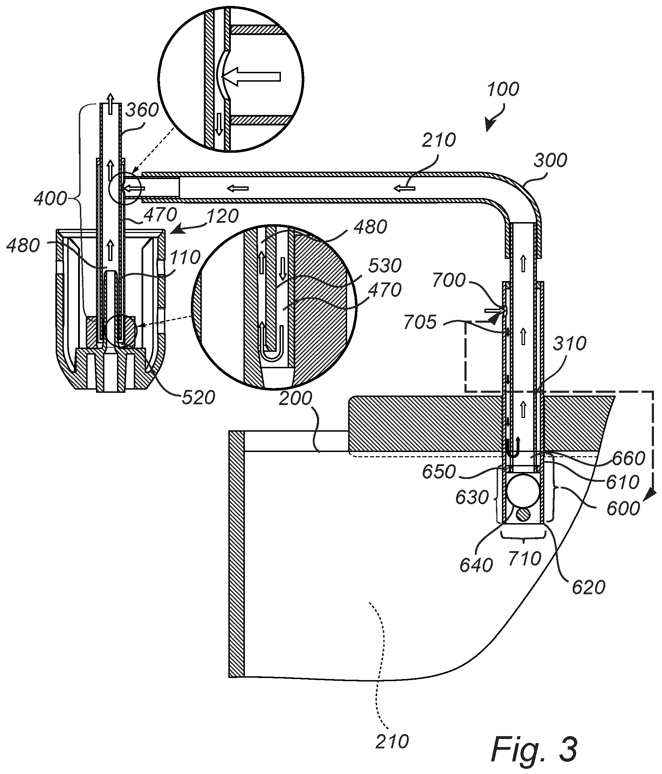

Fig. 3 is a schematic view of a cross-section of an arrangement 100 according to an exemplifying embodiment of the present invention. Compared to Fig. 2, in which the coupling device 120 and the arrangement 100 are separated, the arrangement 100 in Fig. 3 has received one or more components 110 of the coupling device 120 via the opening 520 of the conduit section 400 of the conduit 300. More specifically, according to the example of Fig. 3, the component 110 of the coupling device 120 is a tube, and the opening 520 has received the tube by a male/female connection in a mating manner at the state of the arrangement 100 and coupling device 120 for lubrication of (at least a portion of) the tube/component 110. According to an example, the conduit section 400 may comprise a sealing (not shown) at a peripheral portion of the conduit section 400 defining the opening 520, for a sealing of the reception of the component(s) 110 (e.g. the tube) of the coupling device 120 to the opening 520.

Via the underpressure as generated by the pump (not shown) of the arrangement 100, the lubricant 210 is transferred through the conduit 300 to the opening 520 of the conduit section 400. The conduit section 400, arranged between the first end portion 310 and the second end portion 360 of the conduit 300, comprises a first passage 470 for the lubricant 210. The first passage 470 is fluidically connected to the opening 520 and arranged upstream of the opening 520. Hence, and according to the example of Fig. 3, the lubricant 210 is arranged to flow vertically downwards through the first passage 470 to the opening 520. The conduit section 400 further comprises a second passage 480 for the lubricant, wherein the second passage 480 is fluidically connected to the opening 520 and arranged downstream of the opening 520. The parts/portions of the first and second passages 470, 480 in a vicinity of the opening 520 are shown in the zoomed-in (circular) figure in Fig. 3. Hence, and according to the example of Fig. 3, the lubricant 210 is arranged to flow vertically upwards from the opening 520 through the second passage 480. According to the example of the arrangement 100 of Fig. 3, the first passage 470 and the second passage 480 are separated by a wall portion 530.

The arrangement 100 for lubrication as shown in Fig. 3 further discloses a regulator mechanism 600 for regulating the supply of lubricant 210 from the container 200 to the conduit 300. The regulator mechanism 600 comprises an outer tube 610 at least partially enclosing the first end portion 310 of the conduit 300, wherein a portion 620 of the outer tube 610 extends into the container 200. The regulator mechanism 600 further comprises a floater arrangement 630 arranged in the container 200 and within the portion 620 of the outer tube 610. The floater arrangement 630 comprises a floater 640, which is exemplified as a sphere with a diameter which is marginally smaller than the diameter of the portion 620 of the outer tube 610, thereby allowing a (vertical) motion of the floater 640 within the portion 620 of the outer tube 610. The floater arrangement 630 further comprises a sealing abutment 650 for the floater 640. Here, the sealing abutment 650 is exemplified as a ring with a smaller diameter than the floater 640. The sealing abutment 650 defines a space 660 of the portion 620 of the outer tube 610, i.e. a space 660 which is defined above the sealing abutment 650. The outer tube 610 comprises at least one aperture 700 allowing a passage of ambient air 705 outside the outer tube 610 to the space 660 of the portion 620 of the outer tube 610. Hence, the aperture(s) (and/or hole(s)) 700 is arranged or configured to let a flow or passage of ambient air 705 pass within the outer tube 610 (e.g. in a passage defined by the first end portion 310 and the outer tube 610) to the space 660. The aperture(s) 700 is (are) part of the regulator mechanism 600, which is indicated by the dashed line in Fig. 3.

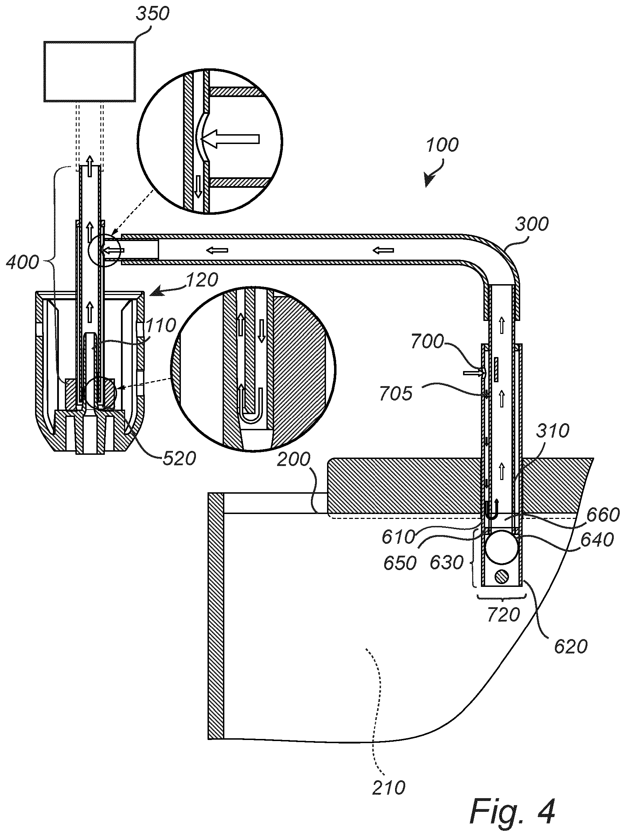

The floater arrangement 630 is operable between a first state 710, as shown in Fig. 3, and a second state 720, as shown in Fig. 4. In the first state 710 of the floater arrangement 630 shown in Fig. 3, the floater 640 is separated from the sealing abutment 650. Hence, there is a (vertical) distance between the floater 640 and the sealing abutment 650. In this first state 710, the arrangement 100 allows or provides a supply of lubricant 210 from the container 200 to the space 660 of the portion 620 of the outer tube 610.

It will be appreciated that Fig. 4 at least partially corresponds to Fig. 3, and it is also referred to Fig. 3 for an increased understanding of the arrangement 100. In Fig. 4, upon a reception of the at least one component 110 of the coupling device 120 to the opening 520 of the arrangement 100, the floater arrangement 630 is operable in its second state 720. Hence, in the second state 720 of the floater arrangement 630, caused by the component 110 reception and the underpressure in the conduit 300 generated by the pump 350 of the arrangement 100, the floater 640 abuts the sealing abutment 650, impeding a supply of lubricant 210 from the container 200 to the space 660 of the portion 620 of the outer tube 610. Hence, in this second state 720, the floater 640 and the sealing abutment 650 provide a sealing such that any supply of lubricant 210 from the container 200 to the space 660 is impeded or hindered. In this second state 720, the floater arrangement 630 defines a predetermined amount of lubricant 210 in the space 660 of the portion 620 of the outer tube 610. The at least one aperture 700 allows ambient air 705 to transfer the predetermined amount of lubricant 210 to the conduit 300. More specifically, the arrangement 100 achieves a significant pressure difference between the ambient air 705 of relatively high pressure, on the one hand, and the conduit 300 and/or conduit section 400 thereof, on the other hand, via the pump 350 creating the underpressure. Hence, by the access of the ambient air 705 into the conduit 300 via the outer tube 610 and the first end portion 310 of the conduit 300, and the underpressure in the conduit 300 and/or conduit section 400 as generated by the pump 350, the predetermined amount of lubricant 210 in the space 660 is transferred to the conduit 300, and further to the conduit section 400, for lubrication of the component(s) 110 of the coupling device 120.

It should be noted that the arrangement 100 may comprise any mechanism, configuration, or the like for adjustment of the amount of lubricant 210 needed for the lubrication of the component(s) 110 of the coupling device 120. For example, the arrangement 100 may configured such that the outer tube 610 is adjustable with respect to the container 200 (e.g. vertically) for an adjustment of the (volume of the) space 660 of the portion 620 of the outer tube 610, and accordingly, and adjustment of the predetermined amount of lubricant 210 in the space 660 for subsequent transfer to the conduit section 400, for lubrication of the component(s) 110 of the coupling device 120.

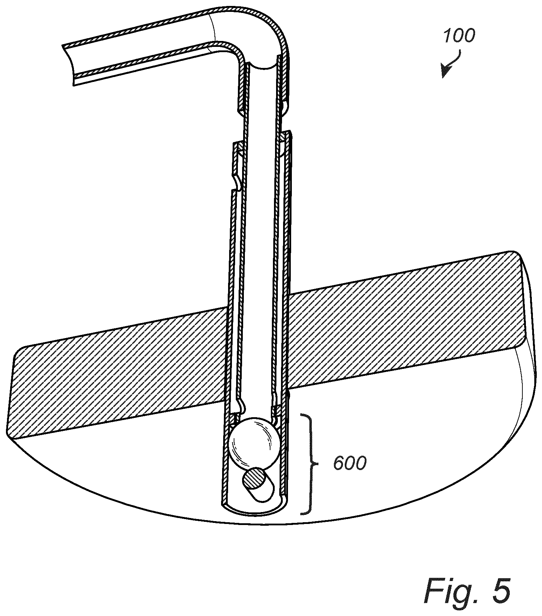

Fig. 5 is a schematic view of a cross-section of a part of the arrangement 100 comprising the regulator mechanism 600. Fig. 5 is provided for the purpose of an increased understanding of the arrangement 100 and/or regulator mechanism 600 described by Fig. 2 and/or Fig. 3 and the associated text(s).

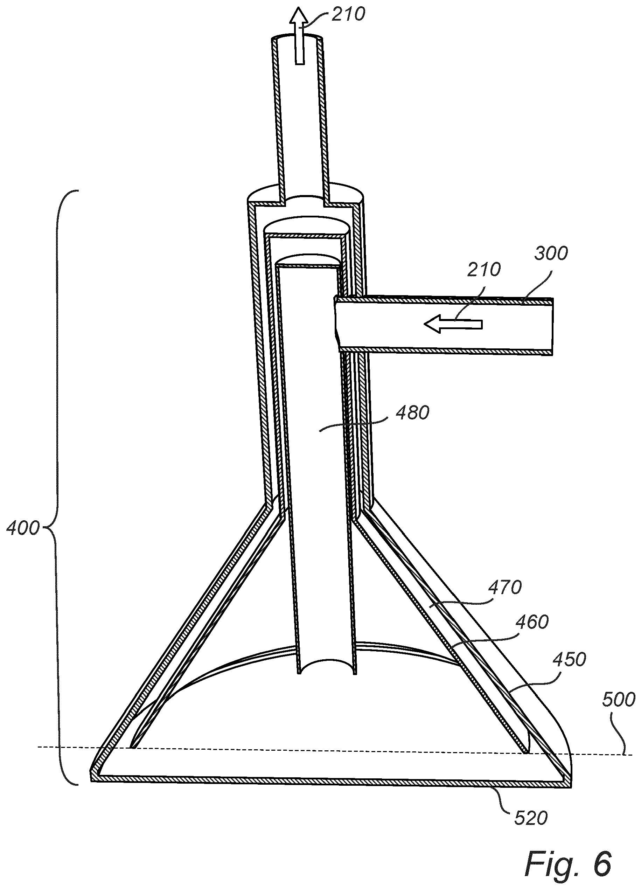

Fig. 6 is a schematic view of a cross-section of a conduit section 400 of an arrangement according to an exemplifying embodiment of the present invention. The conduit section 400 as exemplified in Fig. 6 and the conduit section 400 as exemplified in Fig. 2 have the common concept of being configured or adapted to receive one or more components of a coupling device via the opening 520 of the conduit section 400, whereby the lubricant 210 is transferred through the conduit 300 to the opening via the first passage 470, and from the opening via the second passage 480, for lubrication of the coupling device 120 component(s) 110 as disclosed in Fig. 2. In Fig. 5, the conduit section 400 is funnel-shaped towards its opening 520. In other words, the cross-section of the conduit section 400 increases (in a (downwards) vertical direction) towards the opening 520. The conduit section 400 comprises an outer wall 450 and an inner wall 460 defining the first passage 470, wherein the outer wall 450 defines the opening 520 of the conduit section 400. Albeit the opening 520 of the conduit section 400 as shown in Fig. 5 has a rectangular or quadratic cross-section 500 as indicated by the dashed line, it should be noted that the cross-section of the opening 520 may have substantially any shape. For example, the cross-section of the opening 520 may be circular, oval, or any other shape adapted for the shape and/or size of the component(s) of the coupling device which is (are) to be lubricated by the arrangement 100. The conduit section 400 may be manufactured by 3D-printing.

The person skilled in the art realizes that the present invention by no means is limited to the preferred embodiments described above. On the contrary, many modifications and variations are possible within the scope of the appended claims. For example, it will be appreciated that the figures are merely schematic views of coupling systems according to embodiments of the present invention. Hence, any elements/components of the coupling systems and/or coupling device thereof may have different dimensions, shapes and/or sizes than those depicted and/or described.

## Claims

1. An arrangement (100) for lubrication of at least one component (110), wherein the arrangement comprises
a container (200) arranged to contain a lubricant (210),
a conduit (300) for the lubricant, wherein the conduit is connected to the container at a first end portion (310) of the conduit,
a regulator mechanism (600) for regulating the supply of lubricant from the container to the conduit,
a pump (350) connected to a second end portion (360) of the conduit, wherein the pump, upon operation, is configured to generate an underpressure in the conduit,
wherein the conduit comprises
a conduit section (400) between the first end portion and the second end portion of the conduit, wherein the conduit section comprises
an opening (520) arranged to receive at least a part of the at least one component,
a first passage (470) for the lubricant, wherein the first passage is fluidically connected to the opening and arranged upstream of the opening, and
a second passage (480) for the lubricant, wherein the second passage is fluidically connected to the opening and arranged downstream of the opening,
such that upon a reception of the at least one component to the opening, the lubricant, via the underpressure, is transferred through the conduit to the opening via the first passage, and from the opening via the second passage, for lubrication of the at least one component.

2. The arrangement according to claim 1, wherein the conduit section comprises an outer wall (450) and an inner wall (460) defining the first passage, wherein the outer wall defines the opening of the conduit section.

3. The arrangement according to claim 1 or 2, wherein a cross-section (500) of the opening of the conduit section has one of
a circular shape, and
a rectangular shape.

4. The arrangement according to any one of the preceding claims, wherein the conduit section, towards the opening thereof, has a funnel shape.

5. The arrangement according to claim 1, for lubrication of at least one component constituting a tube, wherein the second passage, via the opening, is arranged to matingly receive at least a part of the tube.

6. The arrangement according to any one of the preceding claims, wherein the conduit section comprises a sealing at a peripheral portion of the conduit section defining the opening, for a sealing of the reception of the at least one component to the opening.

7. The arrangement according to any one of the preceding claims, wherein the pump is a vacuum pump.

8. The arrangement according to any one of the preceding claims, wherein the regulator mechanism comprises
an outer tube (610) at least partially enclosing the first end portion of the conduit, wherein a portion (620) of the outer tube extends into the container,
a floater arrangement (630) arranged in the container and within the portion of the outer tube, wherein the floater arrangement comprises
a floater (640), and
a sealing abutment (650) for the floater, wherein the sealing abutment defines a space (660) of the portion of the outer tube,
wherein the outer tube comprises at least one aperture (700) allowing a passage of ambient air (705) outside the outer tube to the space of the portion of the outer tube, and
wherein the floater arrangement is operable between
a first state (710) of the floater arrangement, wherein the floater is separated from the sealing abutment, allowing a supply of lubricant from the container to the space of the portion of the outer tube, and
a second state (720) of the floater arrangement, caused by a reception of the at least one component to the opening and the underpressure in the conduit, wherein the floater abuts the sealing abutment, impeding a supply of lubricant from the container to the space of the portion of the outer tube,
wherein, in the second state,
the floater arrangement defines a predetermined amount of lubricant in the space of the portion of the outer tube, and the at least one aperture allows ambient air to transfer the predetermined amount of lubricant to the conduit for lubrication of the at least one component.

9. The arrangement according to claim 8, wherein the outer tube is adjustable with respect to the container for an adjustment of the predetermined amount of lubricant in the space of the portion of the outer tube.

10. The arrangement according to claim 8 or 9, further comprising a control unit connected to the regulator mechanism, wherein the control unit is configured to operate the regulator mechanism based on time control.

11. The arrangement according to any one of the preceding claims, wherein the arrangement is arranged for lubrication of at least one component of a coupling device (120), wherein the coupling device is configured to transfer a fluid.

12. A lubrication system (900), comprising
a coupling device configured to transfer a fluid, and
an arrangement according to any one of the preceding claims for lubrication of at least one component of the coupling device.

13. Method for lubrication of at least one component, via an arrangement comprising
a container (200) arranged to contain a lubricant (210),
a conduit (300) for the lubricant, wherein the conduit is connected to the container at a first end portion (310) of the conduit,
a regulator mechanism (600) for regulating the supply of lubricant from the container to the conduit,
a pump (350) connected to a second end portion (360) of the conduit, wherein the pump, upon operation, is configured to generate an underpressure in the conduit,
wherein the conduit comprises
a conduit section (400) between the first end portion and the second end portion of the conduit, wherein the conduit section comprises
an opening (520) arranged to receive at least a part of the at least one component,
a first passage (470) for the lubricant, wherein the first passage is fluidically connected to the opening and arranged upstream of the opening, and
a second passage (480) for the lubricant, wherein the second passage is fluidically connected to the opening and arranged downstream of the opening,
the method comprising the step of
arranging the at least one component at the opening,
whereby the lubricant, via the underpressure, is transferred through the conduit to the opening via the first passage, and from the opening via the second passage, for lubrication of the at least one component.

14. The method according to claim 13, arranged for lubrication of at least one component of a coupling device (120), wherein the coupling device is configured to transfer a fluid.

## Patentansprüche

1. Anordnung (100) zur Schmierung von mindestens einer Komponente (110), wobei die Anordnung umfasst:
einen Behälter (200), der dazu angeordnet ist, ein Schmiermittel (210) zu enthalten,
eine Leitung (300) für das Schmiermittel, wobei die Leitung an einem ersten Endabschnitt (310) der Leitung mit dem Behälter verbunden ist,
einen Regulatormechanismus (600) zum Regulieren der Zufuhr von Schmiermittel aus dem Behälter zur Leitung,
eine Pumpe (350), die mit einem zweiten Endabschnitt (360) der Leitung verbunden ist, wobei die Pumpe, bei Betätigung, dazu ausgelegt ist, einen Unterdruck in der Leitung zu erzeugen,
wobei die Leitung umfasst
einen Leitungsbereich (400) zwischen dem ersten Endabschnitt und dem zweiten Endabschnitt der Leitung, wobei der Leitungsbereich umfasst
eine Öffnung (520), die dazu angeordnet ist, mindestens einen Teil der mindestens einen Komponente aufzunehmen,
einen ersten Durchgang (470) für das Schmiermittel, wobei der erste Durchgang fluidisch mit der Öffnung verbunden und der Öffnung vorgelagert angeordnet ist, und
einen zweiten Durchgang (480) für das Schmiermittel, wobei der zweite Durchgang fluidisch mit der Öffnung verbunden und der Öffnung nachgelagert angeordnet ist,
so dass bei Aufnahme der mindestens einen Komponente an der Öffnung das Schmiermittel über den Unterdruck durch die Leitung über den ersten Durchgang zur Öffnung transferiert wird, und von der Öffnung über den zweiten Durchgang, zur Schmierung der mindestens einen Komponente.

2. Anordnung nach Anspruch 1, wobei der Leitungsbereich eine Außenwand (450) und eine Innenwand (460) umfasst, die den ersten Durchgang definieren, wobei die Außenwand die Öffnung des Leitungsbereichs definiert.

3. Anordnung nach Anspruch 1 oder 2, wobei ein Querschnitt (500) der Öffnung des Leitungsbereichs eine aufweist von
einer kreisförmigen Form, und
einer rechtwinkligen Form.

4. Anordnung nach einem der vorhergehenden Ansprüche, wobei der Leitungsbereich in Richtung der Öffnung davon eine Trichterform aufweist.

5. Anordnung nach Anspruch 1 zur Schmierung von mindestens einer Komponente, die ein Rohr bildet, wobei der zweite Durchgang über die Öffnung dazu angeordnet ist, mindestens einen Teil des Rohres passend aufzunehmen.

6. Anordnung nach einem der vorhergehenden Ansprüche, wobei der Leitungsbereich eine Dichtung an einem peripheren Abschnitt des Leitungsbereichs umfasst, der die Öffnung definiert, für eine Abdichtung der Aufnahme der mindestens einen Komponente an der Öffnung.

7. Anordnung nach einem der vorhergehenden Ansprüche, wobei die Pumpe eine Vakuumpumpe ist.

8. Anordnung nach einem der vorhergehenden Ansprüche, wobei der Regulatormechanismus umfasst
ein Außenrohr (610), das mindestens teilweise den ersten Endabschnitt der Leitung umschließt, wobei sich ein Abschnitt (620) des Außenrohres in den Behälter erstreckt,
eine Schwimmeranordnung (630), die in dem Behälter und in dem Abschnitt des Außenrohres angeordnet ist, wobei die Schwimmeranordnung umfasst
einen Schwimmer (640), und
eine Dichtanlage (650) für den Schwimmer, wobei die Dichtanlage einen Raum (660) des Abschnitts des Außenrohres definiert,
wobei das Außenrohr mindestens einen Durchlass (700) umfasst, der einen Durchgang von Umgebungsluft (705) außerhalb des Außenrohres an den Raum des Abschnitts des Außenrohres erlaubt, und
wobei die Schwimmeranordnung betätigbar ist zwischen
einem ersten Zustand (710) der Schwimmeranordnung, in dem der Schwimmer von der Dichtanlage getrennt ist, was eine Zufuhr von Schmiermittel aus dem Behälter an den Raum des Abschnitts des Außenrohres erlaubt, und
einem zweiten Zustand (720) der Schwimmeranordnung, der durch eine Aufnahme der mindestens einen Komponente an der Öffnung und den Unterdruck in der Leitung bewirkt wurde,
wobei der Schwimmer an der Dichtanlage anliegt, was eine Zufuhr von Schmiermittel aus dem Behälter an den Raum des Abschnitts des Außenrohres verhindert,
wobei im zweiten Zustand
die Schwimmeranordnung eine vorbestimmte Menge von Schmiermittel in dem Raum des Abschnitts des Außenrohres definiert und der mindestens eine Durchlass erlaubt, dass Umgebungsluft die vorbestimmte Menge an Schmiermittel zur Schmierung der mindestens einen Komponente zur Leitung transferiert.

9. Anordnung nach Anspruch 8, wobei das Außenrohr mit Bezug auf den Behälter für eine Einstellung der vorbestimmten Menge an Schmiermittel in dem Raum des Abschnitts des Außenrohres einstellbar ist.

10. Anordnung nach Anspruch 8 oder 9, ferner umfassend eine Steuereinheit, die mit dem Regulatormechanismus verbunden ist, wobei die Steuereinheit dazu ausgelegt ist, den Regulatormechanismus basierend auf Zeitsteuerung zu betätigen.

11. Anordnung nach einem der vorhergehenden Ansprüche, wobei die Anordnung zur Schmierung von mindestens einer Komponente einer Kopplungsvorrichtung (120) angeordnet ist, wobei die Kopplungsvorrichtung dazu ausgelegt ist, ein Fluid zu transferieren.

12. Schmiersystem (900), umfassend
eine Kopplungsvorrichtung, die dazu ausgelegt ist, ein Fluid zu transferieren, und
eine Anordnung nach einem der vorhergehenden Ansprüche zur Schmierung von mindestens einer Komponente der Kopplungsvorrichtung.

13. Verfahren zur Schmierung von mindestens einer Komponente über eine Anordnung, umfassend
einen Behälter (200), der dazu angeordnet ist, ein Schmiermittel (210) zu enthalten,
eine Leitung (300) für das Schmiermittel, wobei die Leitung an einem ersten Endabschnitt (310) der Leitung mit dem Behälter verbunden ist,
einen Regulatormechanismus (600) zum Regulieren der Zufuhr von Schmiermittel aus dem Behälter zur Leitung,
eine Pumpe (350), die mit einem zweiten Endabschnitt (360) der Leitung verbunden ist, wobei die Pumpe, bei Betätigung, dazu ausgelegt ist, einen Unterdruck in der Leitung zu erzeugen,
wobei die Leitung umfasst
einen Leitungsbereich (400) zwischen dem ersten Endabschnitt und dem zweiten Endabschnitt der Leitung, wobei der Leitungsbereich umfasst
eine Öffnung (520), die dazu angeordnet ist, mindestens einen Teil der mindestens einen Komponente aufzunehmen,
einen ersten Durchgang (470) für das Schmiermittel, wobei der erste Durchgang fluidisch mit der Öffnung verbunden und der Öffnung vorgelagert angeordnet ist, und
einen zweiten Durchgang (480) für das Schmiermittel, wobei der zweite Durchgang fluidisch mit der Öffnung verbunden und der Öffnung nachgelagert angeordnet ist, wobei das Verfahren den Schritt umfasst:
Anordnen der mindestens einen Komponente an der Öffnung, wodurch das Schmiermittel, über den Unterdruck, durch die Leitung über den ersten Durchgang zu der Öffnung transferiert wird, und von der Öffnung über den zweiten Durchgang, zur Schmierung der mindestens einen Komponente.

14. Verfahren nach Anspruch 13, das zur Schmierung von mindestens einer Komponente einer Kopplungsvorrichtung (120) angeordnet ist, wobei die Kopplungsvorrichtung dazu ausgelegt ist, ein Fluid zu transferieren.

## Revendications

1. Agencement (100) pour la lubrification d'au moins un composant (110), l'agencement comprenant
un récipient (200) agencé pour contenir un lubrifiant (210),
un conduit (300) pour le lubrifiant, le conduit étant raccordé au récipient au niveau d'une première partie extrémité (310) du conduit,
un mécanisme régulateur (600) pour réguler l'alimentation en lubrifiant depuis le récipient vers le conduit,
une pompe (350) raccordée à une seconde partie extrémité (360) du conduit, la pompe, pendant le fonctionnement, étant configurée pour générer une dépression dans le conduit,
dans lequel le conduit comprend
une section de conduit (400) entre la première partie extrémité et la seconde partie extrémité du conduit, la section de conduit comprenant
une ouverture (520) agencée pour recevoir au moins une partie de l'au moins un composant,
un premier passage (470) pour le lubrifiant, le premier passage étant raccordé fluidiquement à l'ouverture et agencé en amont de l'ouverture, et
un second passage (480) pour le lubrifiant, le second passage étant raccordé fluidiquement à l'ouverture et agencé en aval de l'ouverture, et
de telle sorte que lors d'une réception de l'au moins un composant dans l'ouverture, le lubrifiant, par le biais de la dépression, soit transféré à travers le conduit vers l'ouverture par le biais du premier passage, et de l'ouverture par le biais du second passage, pour la lubrification de l'au moins un composant.

2. Agencement selon la revendication 1, dans lequel la section de conduit comprend une paroi externe (450) et une paroi interne (460) définissant le premier passage, dans lequel la paroi externe définit l'ouverture de la section de conduit.

3. Agencement selon la revendication 1 ou 2, dans lequel une section transversale (500) de l'ouverture de la section de conduit a l'une
d'une forme circulaire, et
d'une forme rectangulaire.

4. Agencement selon l'une quelconque des revendications précédentes, dans lequel la section de conduit, vers son ouverture, a une forme d'entonnoir.

5. Agencement selon la revendication 1, pour la lubrification d'au moins un composant constituant un tube, dans lequel le second passage, par le biais de l'ouverture, est agencé pour recevoir de manière ajustée au moins une partie du tube.

6. Agencement selon l'une quelconque des revendications précédentes, dans lequel la section de conduit comprend un joint étanche au niveau d'une partie périphérique de la section de conduit définissant l'ouverture, pour l'étanchéité de la réception de l'au moins un composant dans l'ouverture.

7. Agencement selon l'une quelconque des revendications précédentes, dans lequel la pompe est une pompe à vide.

8. Agencement selon l'une quelconque des revendications précédentes, dans lequel le mécanisme régulateur comprend
un tube externe (610) renfermant au moins partiellement la première partie extrémité du conduit, dans lequel une partie (620) du tube externe s'étend à l'intérieur du récipient,
un agencement de flotteur (630) agencé dans le récipient et à l'intérieur de la partie du tube externe, dans lequel l'agencement de flotteur comprend
un flotteur (640), et
une butée d'étanchéité (650) pour le flotteur, dans lequel la butée d'étanchéité définit un espace (660) de la partie du tube externe,
dans lequel le tube externe comprend au moins une ouverture (700) permettant un passage d'air ambiant (705) à l'extérieur du tube externe vers l'espace de la partie du tube externe, et
dans lequel l'agencement de flotteur peut fonctionner entre
un premier état (710) de l'agencement de flotteur, dans lequel le flotteur est séparé de la butée d'étanchéité, permettant une alimentation en lubrifiant du récipient vers l'espace de la partie du tube externe, et
un second état (720) de l'agencement de flotteur, provoqué par une réception de l'au moins un composant dans l'ouverture et la dépression dans le conduit,
dans lequel le flotteur bute contre la butée d'étanchéité, empêchant une alimentation en lubrifiant depuis le récipient vers l'espace de la partie du tube externe,
dans lequel, dans le second état,
l'agencement de flotteur définit une quantité prédéterminée de lubrifiant dans l'espace de la partie du tube externe, et l'au moins une ouverture permet à l'air ambiant de transférer la quantité prédéterminée de lubrifiant vers le conduit pour la lubrification de l'au moins un composant.

9. Agencement selon la revendication 8, dans lequel le tube externe est réglable par rapport au récipient pour un ajustement de la quantité prédéterminée de lubrifiant dans l'espace de la partie du tube externe.

10. Agencement selon la revendication 8 ou 9, comprenant en outre une unité de commande connectée au mécanisme régulateur, dans lequel l'unité de commande est configurée pour actionner le mécanisme régulateur sur la base d'une commande temporelle.

11. Agencement selon l'une quelconque des revendications précédentes, l'agencement étant agencé pour la lubrification d'au moins un composant d'un dispositif d'accouplement (120), dans lequel le dispositif d'accouplement est configuré pour transférer un fluide.

12. Système de lubrification (900), comprenant :
un dispositif d'accouplement conçu pour transférer un fluide, et
un agencement selon l'une quelconque des revendications précédentes pour la lubrification d'au moins un composant du dispositif d'accouplement.

13. Procédé pour la lubrification d'au moins un composant, par le biais d'un agencement comprenant
un récipient (200) agencé pour contenir un lubrifiant (210),
un conduit (300) pour le lubrifiant, le conduit étant raccordé au récipient au niveau d'une première partie extrémité (310) du conduit,
un mécanisme régulateur (600) pour réguler l'alimentation en lubrifiant depuis le récipient vers le conduit,
une pompe (350) raccordée à une seconde partie extrémité (360) du conduit, la pompe, pendant le fonctionnement, étant configurée pour générer une dépression dans le conduit,
dans lequel le conduit comprend
une section de conduit (400) entre la première partie extrémité et la seconde partie extrémité du conduit, la section de conduit comprenant
une ouverture (520) agencée pour recevoir au moins une partie de l'au moins un composant,
un premier passage (470) pour le lubrifiant, le premier passage étant raccordé fluidiquement à l'ouverture et agencé en amont de l'ouverture, et
un second passage (480) pour le lubrifiant, le second passage étant raccordé fluidiquement à l'ouverture et agencé en aval de l'ouverture, et
le procédé comprenant l'étape
d'agencement de l'au moins un composant au niveau de l'ouverture,
moyennant quoi le lubrifiant, par l'intermédiaire de la dépression, est transféré à travers le conduit vers l'ouverture par le biais du premier passage, et depuis l'ouverture par le biais du second passage, pour la lubrification de l'au moins un composant.

14. Procédé selon la revendication 13, agencé pour la lubrification d'au moins un composant d'un dispositif d'accouplement (120), dans lequel le dispositif d'accouplement est configuré pour transférer un fluide.
